(19) Europäisches Patentamt
European Patent Office
Office européen des brevets

(11) Veröffentlichungsnummer : **0 078 003**
**B1**

(12) **EUROPÄISCHE PATENTSCHRIFT**

(45) Veröffentlichungstag der Patentschrift : **05.03.86**

(51) Int. Cl.⁴ : **C 07 D513/04**, A 01 N 43/90 // (C07D513/04, 277:00, 239:00)

(21) Anmeldenummer : **82109665.8**

(22) Anmeldetag : **20.10.82**

(54) **Thiazolo(2,3-b)-chinazolone und Verfahren zu ihrer Herstellung.**

(30) Priorität : **28.10.81 DE 3142727**

(43) Veröffentlichungstag der Anmeldung : **04.05.83 Patentblatt 83/18**

(45) Bekanntmachung des Hinweises auf die Patenterteilung : **05.03.86 Patentblatt 86/10**

(84) Benannte Vertragsstaaten : **AT BE CH DE FR GB IT LI NL**

(56) Entgegenhaltungen :
**EP-A- 0 006 114**
**EP-A- 0 027 268**
**FR-A- 2 393 001**
**PHARMAZIE, Band 34, Nr. 4, 1979, Seiten 209-213, Berlin, DE. G. WAGNER et al.: "Synthese kupplungsfähiger 2,3-Dihydroimidazo(1,2-c)-5-Oxo-2,3,5,6-tetrahydromidazo(1,2-c)-und 5-Oxo-1,2,3,5- tetrahydroimidazo(2,1-b)chinazoline mit Isothiocyanatstruktur"**
**CHEMICAL ABSTRACTS, Band 73, 1970, Seite 359, Nr. 130960f, Columbus, Ohio, USA S.K. MODI et al.: "Thiopegan derivatives. XLIII. 2,3-Diaryl-10,11-thiopegan derivatives"**
**CENTRAL PATENTS INDEX, Basic Abstracts Journal, Section C: Agdoc, Woche B08, 18. April 1979, Zusammenfassung Nr. 14891B/08, London, G.B.**

(73) Patentinhaber : **BASF Aktiengesellschaft**
**Carl-Bosch-Strasse 38**
**D-6700 Ludwigshafen (DE)**

(72) Erfinder : **Seybold, Guenther, Dr.**
**Friedrich-Ebert-Strasse 14**
**D-6708 Neuhofen (DE)**
Erfinder : **Wuerzer, Bruno, Dr. Dipl.-Landwirt**
**Ruedigerstrasse 13**
**D-6701 Otterstadt (DE)**

**EP 0 078 003 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft Thiazolo [2,3-b]-chinazolone sowie Verfahren zu ihrer Herstellung.

Thiazolo [2,3-b]-chinazolone mit antibakterieller und pharmazeutischer Wirkung sind bekannt (J. Sci. Ind. Research (1958) 17 B, 120-123 ; DE-OS 25 57 425).

Es wurden neue Thiazolo [2,3-b]-chinazolone der Formel

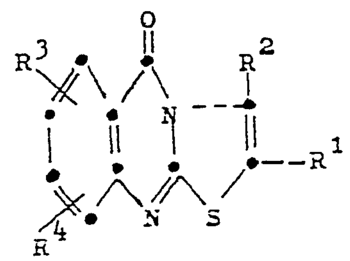

(I)

gefunden, in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Hydroxyalkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-4-yl, 2-Methyl-thien-4-yl oder 1-Methyl-benzimidazol-2-yl und

$R^3$ und $R^4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylamino- oder Di-hydroxyalkylaminosulfonyl mit 1 bis 4 Kohlenstoffatomen in einer Alkylgruppe, Morpholinylsulfonyl, Alkylsulfonylamino mit 1 bis 4 Kohlenstoffatomen, Alkoxycarbonylalkylamino mit 3 bis 6 Kohlenstoffatomen, N-(ω-Dialkylamino-alkyl)-aminosulfonyl mit 1 bis 4 Kohlenstoffatomen in einer Alkylgruppe oder N-(2-Sulfo-ethyl)-amino-sulfonyl bedeuten,

mit der Maßgaben daß $R^3$ Fluor, Nitro, Alkyl mit 2 bis 9 Kohlenstoffatomen, Halogenalkyl, Cycloalkyl, Alkoxy mit 4 bis 9 Kohlenstoffatomen, Alkylthio, Thiophenyl, Amino, Alkylamino, Dialkylamino, Alkanoylamino, Halogenalkanoylamino, Sulfo, Sulfino, Alkylsulfonyl, Alkylsulfinyl, Chlorsulfonyl, Phenylsulfonyl, Sulfamoyl, Alkylaminosulfinyl, Alkylaminosulfonyl, Hydroxyalkylaminosulfonyl, Di-hydroxyalkylaminosulfonyl, Morpholinylsulfonyl, Alkylsulfonylamino oder Alkoxycarbonylalkylamino bedeutet und

$R^4$ die obengenannten Bedeutungen hat, wenn $R^1$ Wasserstoff, Methyl, Ethyl, Halogenmethyl, Aminomethyl, Alkylaminomethyl oder Dialkylaminomethyl und $R^2$ Wasserstoff, Methyl oder Ethyl bedeuten.

Die neuen Verbindungen oder deren Säureadditionssalze besitzen herbizide und wachstumsbeeinflussende Eigenschaften.

Die Substituenten $R^1$, $R^2$, $R^3$ und $R^4$ in Formel I stehen für Wasserstoff, Halogen, wie Fluor, Chlor, Brom, Jod, Nitro, Cyano, Alkylreste, die im Fall $R^1$ und $R^2$ 1 bis 12, vorzugsweise 1 bis 9, insbesondere 1 bis 4 Kohlenstoffatome und im Fall $R^3$ und $R^4$ 1 bis 4 Kohlenstoffatome aufweisen, wie Methyl, Ethyl, n-Propyl, Isopropyl, n-Butyl, i-Butyl, sec-Butyl, tert.-Butyl, Halogenalkylreste mit 1 bis 4 Kohlenstoffatomen, wie Chlormethyl, Fluormethyl, Difluormethyl, Trichlormethyl, Trifluormethyl, Pentafluorethyl, 2-Chlor-1,1,2,2-tetrafluorethyl, Nonafluor-n-butyl, Cycloalkylreste mit 3 bis 6 Kohlenstoffatomen, wie Cyclopropyl, Cyclopentyl, Cyclohexyl, Alkoxy- oder Alkylthioreste mit 1 bis 9, vorzugsweise 1 bis 6, insbesondere 1 bis

4 Kohlenstoffatomen, wie Methoxy, Methylthio, Ethylthio, n-Propylthio, Ethoxy, n-Butoxy, n-Butylthio, Isopropoxy, tert.-Butoxy, für Thiophenyl, Amino, Alkylamino oder Dialkylamino mit 1 bis 4 Kohlenstoffatomen in einer Alkylgruppe, wie Methylamino, Dimethylamino, n-Butylamino, Diethylamino, Isopropylamino, Methylethylamino, Aminoalkyl, Alkylaminoalkyl oder Dialkylaminoalkyl mit jeweils 1 bis 4 Kohlenstoffatomen in einer Alkylgruppe, wie Aminomethyl, Methylaminomethyl, Dimethylaminomethyl, Aminoethyl, Methylaminoethyl, Dimethylaminoethyl, Alkanoylamino oder Halogenalkanoylamino mit 2 bis 5 Kohlenstoffatomen, wie Acetylamino, Chloracetylamino, Trifluoracetylamino, Propionylamino, 2-Chlorpropionylamino, Dialkylcarbamido mit 1 bis 4 Kohlenstoffatomen in einer Alkylgruppe, wie N,N-Dimethylcarbamido, N,N-Diethylcarbamido, N,N-Di-n-butylcarbamido, Alkoxycarbonylalkyl mit 3 bis 6 Kohlenstoffatomen, wie Methoxycarbonylmethyl, Ethoxycarbonylmethyl, n-Butoxycarbonylmethyl, gegebenenfalls durch Alkoxy mit 1 bis 4 Kohlenstoffatomen substituiertes Alkoxycarbonyl mit 2 bis 6 Kohlenstoffatomen, wie Methoxycarbonyl, Ethoxycarbonyl, n-Propoxycarbonyl, n-Butoxycarbonyl, Isopropoxycarbonyl, 2-Methoxyethoxycarbonyl, Ethoxymethoxycarbonyl, 2-Ethoxy-ethoxycarbonyl, Sulfo, Sulfino, Alkylsulfonyl oder Alkylsulfinyl mit 1 bis 4 Kohlenstoffatomen, wie Methylsulfonyl, Methylsulfinyl, Isopropylsulfonyl, n-Propylsulfinyl, n-Butylsulfonyl, n-Butylsulfinyl, Chorsulfonyl, Phenylsulfonyl, Sulfamoyl, Alkylaminosulfonyl oder Alkylaminosulfinyl mit 1 bis 4 Kohlenstoffatomen, wie Methylaminosulfonyl, Methylaminosulfinyl, Isopropylaminosulfonyl, n-Butylaminosulfonyl, n-Butylaminosulfinyl, Hydroxyalkylaminosulfonyl oder Dihydroxyalkylaminosulfonyl mit 1 bis 4 Kohlenstoffatomen in einer Hydroxylalkylgruppe, wie 2-Hydroxyethylaminosulfonyl, Di-(2-hydroxyethyl)aminosulfonyl, Morpholinylsulfonyl, Alkylsulfonylamino mit 1 bis 4 Kohlenstoffatomen, wie Methylsulfonylamino, Isopropylsulfonylamino, Isobutylsulfonylamino oder Alkoxycarbonylalkylamino mit 3 bis 6 Kohlenstoffatomen, wie 2-Methoxycarbonylethylamino, 2-n-Butoxycarbonylethylamino.

Die Substituenten $R^1$ und $R^2$ stehen weiterhin für gegebenenfalls durch Halogen oder Alkyl mit 1 bis 4 Kohlenstoffatomen substituiertes Thien-2-yl, Thiazol-2-yl, Fur-2-yl, Benzimidazol-2-yl, 2-Chlor-thien-4-yl, 2-Methylthien-2-yl oder 1-Methyl-benzimidazol-2-yl.

Die Substituenten $R^3$ und $R^4$ bedeuten außerdem N-(ω-Dialkylamino-alkyl)-aminosulfonyl mit 1 bis 4 Kohlenstoffatomen in einer Alkylgruppe, insbesondere N-(2-Dialkylaminoethyl)-aminosulfonyl, wie N-(2-Diethylamino-ethyl)-amino-sulfonyl, N-(2-Dimethylaminoethyl)-aminosulfonyl oder N-(2-Sulfoethyl)-amino-sulfonyl.

Bevorzugte Verbindungen der Formel I sind solche, bei denen $R^1$ Wasserstoff, Halogen, Alkyl mit 1 bis 4 Kohlenstoffatomen oder Alkoxycarbonyl mit 2 bis 5 Kohlenstoffatomen, $R^2$ Wasserstoff, Chlor, Alkyl mit 1 bis 4 Kohlenstoffatomen oder Alkoxycarbonyl mit 2 bis 5 Kohlenstoffatomen, $R^3$ Fluor oder Sulfo und $R^4$ Wasserstoff bedeuten, oder Säureadditionssalze dieser Verbindungen. Von diesen Verbindungen sind insbesondere solche bevorzugt, bei denen $R^3$ in 6-Stellung steht und Fluor bedeutet, oder solche, bei denen $R^3$ in 7-Stellung steht und Sulfo bedeutet.

Man erhält die Thiazolo [2,3-b]-chinazolone der Formel I durch Umsetzung von Anthranilsäurederivaten der Formel

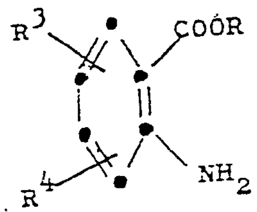

(II)

in der R Wasserstoff oder Alkyl, vorzugsweise mit 1 bis 4 Kohlenstoffatomen, bedeutet und $R^3$ und $R^4$ die oben genannten Bedeutungen haben, mit einem Thiocyanatderivat der Formel

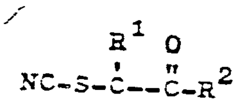

(III)

in der $R^1$ und $R^2$ die obengenannten Bedeutungen haben, oder einem Thiazolderivat der Formel

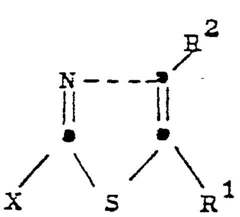

(IV)

in der X Fluor, Chlor, Brom, Alkylsulfonyl oder Arylsulfonyl bedeutet und $R^1$ und $R^2$ die obengenannten Bedeutungen haben.

Die Kondensation des Anthranilsäurederivats der Formel II mit dem Thiocyanatderivat der Formel III wird vorzugsweise in wäßrigem Medium in Gegenwart starker Mineralsäuren, wie Chlorwasserstoff, Bromwasserstoff oder Schwefelsäure, durchgeführt. Die Ausgangstoffe werden vorzugsweise in ungefähr stöchimetrischem Verhältnis eingesetzt ; ein Überschuß der einen oder anderen Reaktionskomponente beeinträchtigt den Reaktionsablauf nicht. Die Reaktionstemperatur kann im Bereich zwischen 40 und 150 °C, vorzugsweise zwischen 60 und 100 °C, variiert werden. Die Säuremenge beträgt 1 bis 2 Moläquivalente, bezogen auf die Menge an Anthranilsäurederivat der Formel II.

Die Umsetzung des Anthranilsäurederivats der Formel II mit dem Thiazolderivat der Formel IV wird bei einer Temperatur im Bereich zwischen 100 und 200 °C, vorzugsweise zwischen 130 und 160 °C, durchgeführt. Die Reaktionskomponenten werden dabei ebenfalls in ungefähr stöchiometrischem Verhältnis eingesetzt. Ein Säurezusatz ist nicht erforderlich. Die Umsetzung kann gegebenenfalls in Gegenwart eines Lösungsmittels durchgeführt werden. In Betracht kommen halogenierte Kohlenwasserstoffe, wie Dichlorbenzol, Trichlorbenzol, mehrwertige Alkohole, wie Glykol, Ethylglykol, Butylglykol, Ester, wie Methylglykolacetat, und Dimethylformamid. Auch Gemische dieser Lösungsmittel können verwendet werden. Vorzugsweise führt man die Umsetzung lösungsmittelfrei durch.

X in Formel IV bedeutet vorzugsweise Methylsulfonyl.

Die als Ausgangsstoffe eingesetzten Anthranilsäurederivate der Formel II, Thiocyanatderivate der Formel III und Thiozolderivate der Formel IV sind zum Teil bekannt ; sie können in Analogie zu bekannten Methoden hergestellt werden (JACS *74*, 1719 (1952) ; Proc. Ind. Acad. Sci. *22A*, 343 (1945)).

Die Säureadditionssalze der Thiazolo [2,3-b]-chinazolone der Formel I erhält man durch Protonierung mit den entsprechenden Säuren in Gegenwart eines inerten Lösungsmittels, wie Tetrahydrofuran, Dioxan, tert.-Butyl-methylether, Methylenchlorid oder Acetonitril.

Als Säuren, die entsprechende Salze bilden, kommen beispielsweise anorganische Säuren, wie Chlorwasserstoffsäure, Bromwasserstoffsäure, Fluorwasserstoffsäure, Schwefelsäure, Perchlorsäure, Phosphorsäure, oder organische Säuren, wie Trichloressigsäure, Trifluoressigsäure, Dichloressigsäure, Monochloressigsäure, in Betracht.

## Beispiel 1

16,9 Teile 6-Fluor-anthranilsäuremethylester werden in 90 Teilen Wasser und 12 Teilen konz. Salzsäure mit 11,5 Teilen Acetonylrhodanid versetzt und 4 Stunden auf 80 °C erwärmt. Das gebildete 3-Methyl-6-fluor-5H-thiazolo [2,3-b]-chinazolin-5-on (18 Teilen) wird abgesaugt und getrocknet ; Schmp. 251-253 °C.

Analyse :

| | | | | | |
|---|---|---|---|---|---|
| Ber. : | C 56,40 | H 2,99 | N 11,96 | S 13,67 | F 8,11 |
| Gef. : | C 56,4 | H 3,2 | N 12,3 | S 13,7 | F 8,2 |

## Beispiel 2

11,7 Teile des nach Beispiel 1 hergestellten Thiazolochinazolons werden in 80 Teilen Tetrahydrofuron heiß gelöst und mit 17,2 Teilen 47 %iger Bromwasserstoffsäure versetzt. Der Niederschlag wird abgesaugt und getrocknet. Man erhält 13 Teile des Hydrobromids von 3-Methyl-6-fluor-5H-thiazolo [2,3-b]-chinazolin-5-on vom Schmp. 284 °C.

Analyse :

Ber. : Br 25,4

Gef. : Br 24,8

Analog zu den Beispielen 1 und 2 werden folgende Thiazolo [2,3-b]-chinazolone der Formel I hergestellt :

| Beispiel Nr. | $R^1$ | $R^2$ | $R^3$ | $R^4$ | Schmp. [°C] |
|---|---|---|---|---|---|
| 3 | H | $CH_3$ | 6-Cl | H | |
| 4 | H | $CH_3$ | H | 9-Cl | 221 |
| 5 | H | $CH_3$ | 7-$CH_3$ | H | 180 |
| 6 | H | $CH_3$ | 7-$CH_3$ | H (.HBr) | >300 |
| 7 | $CH_3$ | $CH_3$ | 6-F | H (.$H_2SO_4$) | >300 |
| 8 | H | $CH_3$ | 7-$NO_2$ | H | 308 |
| 9 | H | $-CH_2COOC_2H_5$ | H | H | 134-136 |
| 10 | H | $-CH_2COOC_2H_5$ | 6-F | H | >300 |

(Fortsetzung)

| Beispiel Nr. | $R^1$ | $R^2$ | $R^3$ | $R^4$ | Schmp. [°C] |
|---|---|---|---|---|---|
| 12 | H | Thien-2-yl | H | H | 271-274 |
| 13 | H | $CH_3$ | 7-$NH_2$ | H | 249 |
| 14 | H | $C_2H_5$ | H | H | 185 |

Beispiel 15

15 Teile 2-Methyl-5H-thiazolo [2,3-b]-chinazolin-5-on werden langsam bei 20 bis 30 °C in 70 Teile Chlorsulfonsäure eingetragen. Man rührt 12 Stunden lang nach und fällt das gebildete 2-Methyl-7-chlorsulfonyl-5H-thiazolo [2,3-b]-chinazolin-5-on mit Eiswasser aus. Schmp. 206-209 °C.

Analyse :
Ber. : Cl 11,3
Gef. : Cl 10,7

Beispiel 16

16 Teile des nach Beispiel 15 hergestellten Sulfochlorids werden in 200 Teilen Toluol mit 20 Teilen Butylamin bei 40 °C 2 Stunden gerührt. Nach Abziehen der Lösungsmittels und Zugabe von Wasser wird der ausgefallene Niederschlag abgesaugt und getrocknet. Man erhält 14 Teile 3-Methyl-7-(n-butylaminosulfonyl)-5H-thiazolo [2,3-b]-chinazolin-5-on vom Schmp. 165-167 °C.

Analog zu den Beispielen 15 und 16 werden folgende Thiazolo [2,3-b]-chinazolone der Formel I erhalten :

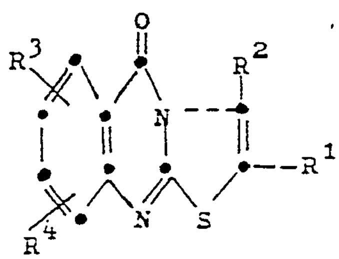

| Beispiel Nr. | $R^1$ | $R^2$ | $R^3$ | $R^4$ | Schmp. [°C] |
|---|---|---|---|---|---|
| 17 | H | $CH_3$ | $(HOC_2H_4)_2N-SO_2-$ | H | 230 |
| 18 | H | $CH_3$ | 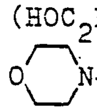-$SO_2$- | H | 266 |
| 20 | H | $CH_3$ | $(C_2H_5)_2N-CH_2-CH_2-NH-SO_2-$ | H | 95-100 |
| 21 | $CH_3$ | $CH_3$ | $ClO_2S-$ | H | 214 |
| 22 | $CH_3$ | $CH_3$ | $CH_3-NH-SO_2-$ | H | |
| 23 | $CH_3$ | $C_2H_5$ | $HOC_2H_4NH-SO_2-$ | H | 210 |
| 24 | $CH_3$ | $C_2H_5$ | $HO_3S-C_2H_4NH-SO_2-$ | H | 305-307 |

Beispiel 25

406 Teile des nach Beispiel 15 hergestellten Sulfochlorids werden in 400 Teilen Wasser mit Natronlauge bei pH 9 verseift. Nach Ansäuern mit Salzsäure fällt die 3-Methyl-7-sulfo-5H-thiazolo [2,3-b]-chinazolin-5-on aus ; sie kann durch Absaugen und Trocknen isoliert werden ; Schmp. 300 °C.

| Beispiel Nr. | $R^1$ | $R^2$ | $R^3$ | $R^4$ | Schmp. [°C] |
|---|---|---|---|---|---|
| 26 | $CH_3$ | $CH_3$ | 7-$SO_3H$ | H | >300 |
| 27 | H | $CH_3$ | 6-F | 7-$SO_3H$ | >300 |
| 28 | H | H | 7-$SO_3H$ | H | >300 |
| 29 | $CH_3$ | $C_2H_5$ | 7-$SO_3H$ | H | >300 |
| 30 | H | $CH_3$ | 7-$SO_3H$ | 9-Cl | >300 |
| 31 | H | $CH_3$ | 7-$SO_3H$ | 9-F | >300 |
| 32 | Cl | $CH_3$ | 7-$SO_3H$ | H | >300 |

Beispiel 34

31,4 Teile des nach Beispiel 15 hergestellten Sulfochlorids werden bei 40 °C in eine Lösung von 18 Teilen Natriumsulfit und 125 Teilen Wasser eingetragen und der pH-Wert wird mit Natriumcarbonat auf 7 gestellt. Dann wird 5 Stunden lang bei 60 °C nachgerührt. Das 3-Methyl-7-sulfino-5H-thiazolo [2,3-b]-chinazolin-5-on läßt sich durch Ansäuern mit Schwefelsäure isolieren ; Schmp. (langsame Zersetzung > 300 °C.

Analog Beispiel 34 können folgende Thiazolo [2,3-b]-chinazolone der Formel I hergestellt werden :

| Beispiel Nr. | $R^1$ | $R^2$ | $R^3$ | $R^4$ | Schmp. [°C] |
|---|---|---|---|---|---|
| 35 | $CH_3$ | $CH_3$ | 7-$SO_2H$ | H | >300 |
| 36 | H | $CH_3$ | 6-F | 7-$SO_2H$ | >300 |
| 37 | H | H | 7-$SO_2H$ | H | >300 |
| 38 | $CH_3$ | $C_2H_5$ | 7-$SO_2H$ | H | >300 |
| 39 | H | $CH_3$ | 7-$SO_2H$ | 9-Cl | >300 |
| 40 | H | $CH_3$ | 7-$SO_2H$ | 9-F | >300 |
| 41 | H | $CH_3$ | 6-Cl | 7-$SO_2H$ | >300 |

Beispiel 42

25,4 Teile 6-Fluoranthranilsäuremethylester werden zusammen mit 18 Teilen 2-Chlorthiazol auf 150 °C erhitzt. Das sich bildende Methanol wird über eine Brücke abdestilliert. Nach 2 Stunden ist die Reaktion beendet. Der feste Rückstand wird neutralisiert und aus Isopropanol umkristallisiert. Man erhält 30 Teile 6-Fluor-5H-thiazolo [2,3-b]-chinazolin-5-on vom Schmp. 205-207 °C.

Analog zu Beispiel 42 werden folgende Thiazolo [2,3-b]-chinazolone der Formel I hergestellt :

| Beispiel [°C] Nr. | $R^1$ | $R^2$ | $R^3$ | $R^4$ | Schmp. |
|---|---|---|---|---|---|
| 43 | H | H | 6-F | H (x HBr) | 290 |
| 44 | H | H | H | 9-F (x $H_2SO_4$) | >300 |
| 45 | H | H | 6-F | H (x $H_2SO_4$) | >300 |
| 46 | H | H | 7-Cl | H (x $HClO_4$) | >300 |
| 47 | -$COOCH_3$ | $CH_3$ | 6-F | H | |
| 48 | -$COOCH_3$ | $CH_3$ | H | 9-F | |

(Fortsetzung)

| Beispiel Nr. | $R^1$ | $R^2$ | $R^3$ | $R^4$ | Schmp. [°C] |
|---|---|---|---|---|---|
| 49 | Cl | H | H | H | |
| 50 | Cl | $CH_3$ | H | H | |
| 51 | F | $CH_3$ | H | H | |
| 52 | $-COOCH_3$ | $-COOCH_3$ | H | H | |
| 53 | H | $-COOCH_3$ | H | H | ($\nu_{CO}$=1720 $cm^{-1}$) |
| 54 | H | -COOH | H | H | |
| 56 | Cl | $CH_3$ | 6-F | H | |
| 57 | Cl | $CH_3$ | 6-Cl | H | |

Die Verbindungen der Formel I oder deren Säureadditionssalze können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungsformen richten sich ganz nach den Verwendungszwecken ; sie sollten in jedem Fall möglichst die feinste Verteilung der erfindungsgemäßen Wirkstoffe gewährleisten.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z. B. Benzol, Toluol, Xylol, Paraffin, Tetrahydronaphthalin, alkylierte Naphthaline oder deren Derivate, z. B. Methanol, Ethanol, Propanol, Butanol, Chloroform, Tetrachlorkohlenstoff, Cyclohexanol, Cyclohexanon, Chlorbenzol, Isophoron, stark polare Lösungsmittel, wie z. B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulvern, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werde, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylenoctylphenoläther, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z. B. Umhüllungs-, Imprägnierungs- und Homogengranulate, können durch Bindung der Wirkstoffe an festen Trägerstoffen hergestellt werden. Feste Trägerstoffe sind Mineralerden wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Die Formulierungen enthalten zwischen 0,1 und 95 Gewichtsprozent, vorzugsweise zwischen 0,5 und 90 Gewichtsprozent, Wirkstoff.

Beispiele für Formulierungen sind :

I. Man vermischt 90 Gewichtsteile der Verbindung gemäß Beispiel 1 mit 10 Gewichtsteilen N-Methyl-α-pyrrolidon und erhält eine Lösung, die zur Anwendung in Form kleinster Tropfen geeignet ist.

II. 20 Gewichtsteile der Verbindung gemäß Beispiel 2 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gewichtsprozent des Wirkstoffs enthält.

III. 20 Gewichtsteile der Verbindung gemäß Beispiel 6 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteile des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

IV. 20 Gewichtsteile der Verbindung gemäß Beispiel 16 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gewichtsprozent des Wirkstoffs enthält.

V. 20 Gewichtsteile der Verbindung gemäß Beispiel 10 werden mit 3 Gewichtsteilen des Natriumsalzes der Diisobutylnaphthalin-α-sulfonsäure, 17 Gewichtsteilen des Natriumsalzes einer Ligninsulfonsäure aus einer Sulfit-Ablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gewichtsprozent des Wirkstoffs enthält.

VI. 3 Gewichtsteile der Verbindung gemäß Beispiel 23 werden mit 97 Gewichtsteilen feinteiligem Kaolin vermischt. Man erhält auf diese Weise ein Stäubemittel, das 3 Gewichtsprozent des Wirkstoffs enthält.

VII. 30 Gewichtsteile der Verbindung gemäß Beispiel 45 werden mit einer Mischung aus 92 Gewichtsteilen pulverförmigen Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 20 Teile der Verbindung gemäß Beispiel 27 werden mit 2 Teilen Calciumsalz der Dodecylbenzolsulfonsäure, 8 Teilen Fettalkohol-polyglykolether, 2 Teilen Natriumsalz eines Phenol-Harnstoff-Formaldehyd-Kondensates und 68 Teilen eines paraffinischen Mineralöls innig vermischt. Man erhält eine stabile ölige Dispersion.

Die Applikation der Wirkstoffe bzw. der Mittel kann im Vorauflaufverfahren oder im Nachauflaufverfahren, vorzugsweise im Nachauflaufverfahren, erfolgen. Dabei können die Mittel auf den Standort aufgebracht werden, bevor die unerwünschten Pflanzen aus den Samen gekeimt oder aus vegetativen Pflanzenteilen ausgetrieben haben, oder sie werden auf die Blätter der unerwünschten Pflanzen und der Kulturpflanzen appliziert. Vorzugsweise werden die neuen Wirkstoffe nach dem Auflaufen der unerwünschten Pflanzen, sowohl auf Kulturflächen als auch auf unbebautem Land, ausgebracht. Sind die Wirkstoffe für die Kulturpflanze weniger verträglich, so können auch Ausbringungstechniken angewandt werden, bei welchen die herbiziden Mittel mit Hilfe der Spritzgeräte so gespritzt werden, daß die Blätter der empfindlichen Kulturpflanzen nach Möglichkeit nicht getroffen werden, während die Wirkstoffe auf die Blätter darunter wachsender unerwünschter Pflanzen oder die unbedeckte Bodenfläche gelangen (post-directed, lay-by).

Die Aufwandmengen an Wirkstoff betragen je nach Jahreszeit und Wachstumsstadium 0,05 bis 15 kg/ha und mehr, vorzugsweise 0,25 bis 5 kg/ha, wobei sich die höheren Dosen besonders zur totalen Bekämpfung von Vegetationen eignen.

Die herbizide und wachstumsbeeinflussende Wirkung der Verbindungen der Formel I wird durch Gewächshausversuche gezeigt:

Als Kulturgefäße dienen Plastikblumentöpfe mit 300 $cm^3$ Inhalt und lehmigen Sand mit etwa 1,5 % Humus als Substrat. Bei dem für die Nachauflaufbehandlung angezogenen Reis wird Torfmull zugesetzt, um ein einwandreies Wachstum zu gewährleisten. Die Samen der Testpflanzen werden nach Arten getrennt flach eingesät. Unmittelbar danach erfolgt bei Vorauflaufbehandlung das Aufbringen der Wirkstoffe auf die Erdoberfläche. Sie werden hierzu in Wasser als Verteilungsmittel suspendiert oder emulgiert und mittels fein verteilender Düsen gespritzt. Die Aufwandmenge beträgt beispielsweise 2,0 kg Wirkstoff/ha. Nach dem Aufbringen der Mittel beregnet man die Gefäße leicht, um Keimung und Wachstum in Gang zu bringen. Danach deckt man die Gefäße mit durchsichtigen Plastikhauben ab, bis die Pflanzen angewachsen sein. Diese Abdeckung bewirkt ein gleichmäßiges Keimen der Testpflanzen, sofern dies nicht durch die Wirkstoffe beeinträchtigt wird.

Zur Nachauflaufanwendung werden entweder direkt gesäte und in den gleichen Gefäßen aufgewachsene Pflanzen oder zunächst als Keimplfanzen getrennt angezogene und einige Tage vor der Behandlung in die Versuchsgefäße verpflanzte Pflanzen verwendet. Je nach Wuchsform haben die Pflanzen zum

Behandlungstermin eine Höhe von etwa 3 bis 15 cm. Die Aufwandmengen variieren je nach Wirkstoff und liegen beispielsweise bei 2,0 oder 3,0 kg Wirkstoff/ha.

Die Versuchsgefäße werden im Gewächshaus aufgestellt, wobei für wärmeliebende Arten wärmere Bereiche (20 bis 30 °C) und für solche gemäßigter Klimate 15 bis 25 °C bevorzugt werden. Die Versuchsperiode erstreckt sich über 3 bis 4 Wochen.

Während dieser Zeit werden die Pflanzen gepflegt, und ihre Reaktion auf die einzelnen Behandlungen wird ausgewertet.

Bewertet wird nach einer Skala von 0 bis 100. Dabei bedeutet 0 keine Schädigung oder normaler Ablauf und 100 kein Aufgang der Pflanzen bzw. völlige Zerstörung zumindest der oberirdischen Teile.

Bei den Testpflanzen handelt es sich um Abutilon theophrasti (Chinesischer Hanf), Amaranthus retroflexus (zurückgekrümmter Fuchsschwanz), Avena sativum (Hafer), Cassia tora, Chenopodium album (Weißer Gänsefuß), Daucus carota (Wilde Möhre), Hordeum vulgare (Gerste), Ipomoea spp. (Prunkwindearten), Lamium pupureum (Rote Taubnessel), Mercurialis annua, Nicandra physaloides (Giftbeere), Oryza sativa (Reis), Rottboellia exaltata, Rumex obtusifolium (Stumpfblättriger Ampfer), Sesbania exaltata (Turibaum), Setaria spp. (Borstenhirsearten), Sida spinosa, Solanum nigrum (Schwarzer Nachtschatten), Veronica persica (Persischer Ehrenpreis), Viola tricolor, Zea mays (Mais).

Bei Vorauflaufanwendung zeigen beispielsweise die Verbindungen Nr. 1 und 2 eine herbizide Wirkung, ohne Getreidearten zu schädigen.

Die Verbindungen Nr. 1, 2, 28 und 42 bekämpfen beispielsweise bei Aufwandmengen von 2,0 bzw. 3,0 kg/ha bei Nachauflaufanwendung unerwünschte Pflanzen in Kulturen, wie z. B. Reis, Hafer, Mais.

In Anbetracht der guten Verträglichkeit der Wirkstoffe und der Vielseitigkeit der Applikationsmethoden können die erfindungsgemäßen Verbindungen oder diese enthaltende Mittel außer bei den in den Gewächshausversuchen getesteten Nutzpflanzen noch in einer weiteren großen Zahl von Kulturpflanzen zur Beseitigung unerwünschten Pflanzenwuchses eingesetzt werden.

In Betracht kommen beispielsweise die folgenden Kulturen :

| Botanischer Name | Deutscher Name |
|---|---|
| Allium cepa | Küchenzwiebel |
| Ananas comosus | Ananas |
| Arachis hypogaea | Erdnuß |
| Asparagus officinalis | Spargel |
| Avena sativa | Hafer |
| Beta vulgaris spp. altissima | Zuckerrübe |
| Beta vulgaris spp. rapa | Futterrübe |
| Beta vulgaris spp. esculenta | Rote Rübe |
| Brassica napus var. napus | Raps |
| Brassica napus var. napobrassica | Kohlrübe |
| Brassica napus var. rapa | Weiße Rübe |
| Brassica rapa var. silvestris | Rübsen |
| Camellia sinensis | Teestrauch |
| Carthamus tinctorius | Saflor - Färberdistel |
| Carya illinoinensis | Pekannußbaum |
| Citrus limon | Zitrone |
| Citrus maxima | Pampelmuse |
| Citrus reticulata | Mandarine |
| Citrus sinensis | Apfelsine, Orange |
| Coffea arabica (Coffea canephora, Coffea liberica) | Kaffee |
| Cucumis melo | Melone |
| Cucumis sativus | Gurke |
| Cynodon dactylon | Bermudagras |

(Fortsetzung)

| Botanischer Name | Deutscher Name |
|---|---|
| Daucus carota | Möhre |
| Elaeis guineensis | Ölpalme |
| Fragaria vesca | Erdbeere |
| Glycine max | Sojabohne |
| Gossypium hirsutum (Gossypium arboreum Gossypium herbaceum Gossypium vitifolium) | Baumwolle |
| Helianthus annuus | Sonnenblume |
| Helianthus tuberosus | Topinambur |
| Hevea brasiliensis | Parakautschukbaum |
| Hordeum vulgare | Gerste |
| Humulus lupulus | Hopfen |
| Ipomoea batatas | Süßkartoffeln |
| Juglans regia | Walnußbaum |
| Lactua sativa | Kopfsalat |
| Lens culinaris | Linse |
| Linum usitatissimum | Faserlein |
| Lycopersiocon lycopersicum | Tomate |
| Malus spp. | Apfel |
| Manihot esculenta | Maniok |
| Medicago sativa | Luzerne |
| Mentha piperita | Pfefferminze |
| Musa spp. | Obst- u. Mehlbanane |
| Nicotiana tabacum (N. rustica) | Tabak |
| Olea europaea | Ölbaum |
| Oryza sativa | Reis |
| Panicum miliaceum | Rispenhirse |
| Phaseolus lunatus | Mondbohne |
| Phaseolus mungo | Erdbohne |
| Phaseolus vulgaris | Buschbohnen |
| Pennisetum glaucum | Perl- oder Rohrkolbenhirse |
| Petroselinum crispum spp. tuberosum | Wurzelpetersilie |
| Picea abies | Rotfichte |
| Abies alba | Weißtanne |
| Pinus spp. | Kiefer |
| Pisum sativum | Gartenerbse |
| Prunus avium | Süßkirsche |
| Prunus domestica | Pflaume |
| Prunus dulcis | Mandelbaum |
| Prunus persica | Pfirsich |

(Fortsetzung)

| Botanischer Name | Deutscher Name |
|---|---|
| Pyrus communis | Birne |
| Ribes sylvestre | Rote Johannisbeere |
| Ribes uva-crispa | Stachelbeere |
| Ricinus communis | Rizinus |
| Saccharum officinarum | Zuckerrohr |
| Secale cereale | Roggen |
| Sesamum indicum | Sesam |
| Solanum tuberosum | Kartoffel |
| Sorghum bicolor (s. vulgare) | Mohrenhirse |
| Sorghum dochna | Zuckerhirse |
| Spinacia oleracea | Spinat |
| Theobroma cacao | Kakaobaum |
| Trifolium pratense | Rotklee |
| Triticum aestivum | Weizen |
| Vaccinium carymbosum | Kulturheidelbeere |
| Vaccinium vitis-idaea | Preißelbeere |
| Vicia faba | Pferdebohnen |
| Vigna sinensis (V. unguiculata) | Kuhbohne |
| Vitis vinifera | Weinrebe |
| Zea mays | Mais |

Zur Verbreiterung des Wirkungsspektrums und zur Erzielung synergistischer Effekte können die neuen erfindungsgemäßen Verbindungen mit zahlreichen Vertretern anderer herbizider oder wachstumsregulierender Wirkstoffgruppen gemischt und gemeinsam ausgebracht werden. Beispielsweise kommen als Mischungspartner Diazine, 4H-3,1-Benzoxazinderivate, Benzothiadiazinone, 2,6-Dinitroaniline, N-Phenylcarbamate, Thiolcarbamate, Halogencarbonsäuren, Triazine, Amide, Harnstoffe, Diphenylether, Triazinone, Uracile, Benzofuranderivate, Cyclohexan-1,3-dionderivate und andere in Betracht.

Außerdem kann es von Nutzen sein, die erfindungsgemäßen Verbindungen allein oder in Kombination mit anderen Herbiziden auch noch mit weiteren Pflanzenschutzmitteln gemischt gemeinsam auszubringen, beispielsweise mit Mitteln zur Bekämpfung von Schädlingen oder phytopathogenen Pilzen bzw. Bakterien. Von Interesse ist ferner die Mischbarkeit mit Mineralsalzlösungen, welche zur Behebung von Ernährungs- oder Spurenelementmängeln eingesetzt werden. Zur Aktivierung der herbiziden Wirkung können auch Netz- und Haftmittel sowie nicht-phytotoxische Öle und Ölkonzentrate zugesetzt werden.

**Patentansprüche** (für die Vertragsstaaten : BE, CH, DE, FR, GB, IT, LI, NL)

1. Thiazolo [2,3-b]-chinazolone der Formel

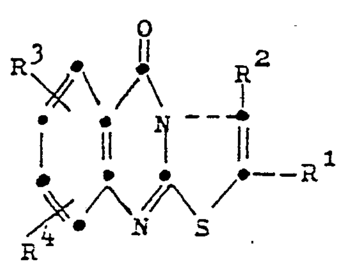

(I)

in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkyklgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Hydroxyalkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-2-yl, 2-Methyl-thien-4-yl oder 1-Methyl-benzimidazol-2-yl und

$R_3$ und $R_4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylamino- oder Di-hydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, N-(ω-Dialkylamino-alkyl)-amino-sulfonyl mit 1 bis 4 Kohlenstoffatomen in einer Alkylgruppe oder N-(2-Sulfoethyl)-amino-sulfonyl bedeuten,

mit der Maßgabe, daß $R^3$ Fluor, Nitro, Alkyl mit 2 bis 9 Kohlenstoffatomen, Halogenalkyl, Cycloalkyl, Alkoxy mit 4 bis 9 Kohlenstoffatomen, Alkylthio, Thiophenyl, Amino, Alkylamino, Dialkylamino, Alkanoylamino, Halogenalkanoylamino, Sulfo, Sulfino, Alkylsulfonyl, Alkylsulfinyl, Chlorsulfonyl, Phenylsulfonyl, Sulfamoyl, Alkylaminosulfinyl, Alkylaminosulfonyl, Hydroxyalkylaminosulfonyl, Di-hydroxyalkylaminosulfonyl, Morpholinylsulfonyl, Alkylsulfonylamino oder Alkoxycarbonylalkylamino bedeutet und

$R_4$ die obengenannten Bedeutungen hat, wenn $R^1$ Wasserstoff, Methyl, Ethyl, Halogenmethyl, Aminomethyl, Alkylaminomethyl oder Dialkylaminomethyl und $R^2$ Wasserstoff, Methyl oder Ethyl bedeuten, oder Säureadditionssalze dieser Verbindungen.

2. Thiazolo [2,3-b]-chinazolone der Formel

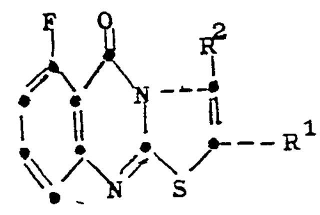

in der $R^1$ Wasserstoff, Halogen, Alkyl mit 1 bis 4 Kohlenstoffatomen oder Alkoxycarbonyl mit 2 bis 5 Kohlenstoffatomen und $R^2$ Wasserstoff, Chlor, Alkyl mit 1 bis 4 Kohlenstoffatomen oder Alkoxycarbonyl mit 2 bis 5 Kohlenstoffatomen bedeuten, und Säureadditionssalze dieser Thiazolo [2,3-b]-chinazolone.

3. Thiazolo [2,3-b]-chinazolone der Formel

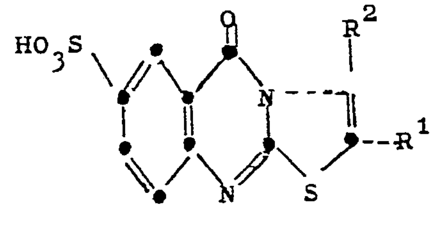

in der $R^1$ Wasserstoff, Halogen, Alkyl mit 1 bis 4 Kohlenstoffatomen oder Alkoxycarbonyl mit 2 bis 5 Kohlenstoffatomen bedeuten und $R^2$ Wasserstoff, Chlor, Alkyl mit 1 bis 4 Kohlenstoffatomen oder

Alkoxycarbonyl mit 2 bis 5 Kohlenstoffatomen, und Säureadditionssalze dieser Thiazolo [2,3-b]-chinazolone.

4. Verfahren zur Herstellung von Thiazolo [2,3-b]-chinazolonen der Formel I gemäß Anspruch 1, dadurch gekennzeichnet, daß man ein Anthranilsäurederivat der Formel

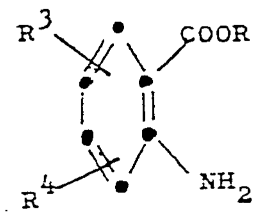

(II)

in der

R Wasserstoff oder Alkyl bedeutet und

$R^3$ und $R^4$ die im Anspruch 1 genannten Bedeutungen haben, mit einem Thiocyanatderivat der Formel

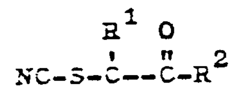

(III)

in der $R^1$ und $R^2$ die im Anspruch 1 genannten Bedeutungen haben, oder mit einem Thiazolderivat der Formel

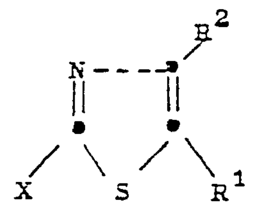

(IV)

in der

X Fluor, Chlor, Brom, Alkylsulfonyl oder Arylsulfonyl bedeutet und

$R^1$ und $R^2$ die im Anspruch 1 genannten Bedeutungen haben,

umsetzt.

**Patentanspruch** (für den Vertragsstaat AT)

Verfahren zur Herstellung von Thiazolo [2,3-b]-chinazolonen der Formel I

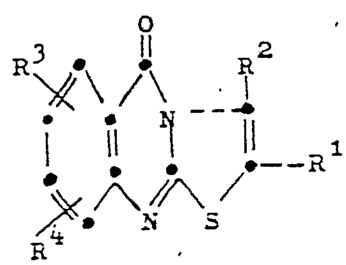

(I)

in der

$R^1$ und $R^2$ unabhängig voneinander Wasserstoff, Halogen, Nitro, Alkyl mit 1 bis 12 Kohlenstoffatomen, Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl,

Alkylaminosulfinyl oder Alkylaminosulfonyl mit 1 bis 4 Kohlenstoffatomen, Hydroxyalkylaminosulfonyl oder Dihydroxyalkylaminosulfonyl mit 1 bis 4 Kohlenstoffatomen in einer Hydroxyalkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, oder gegebenenfalls durch Halogen oder Alkyl mit 1 bis 4 Kohlenstoffatomen einfach substituiertes Thien-2-yl, Thiazol-2-yl, Fur-2-yl, Benzimidazol-2-yl, 2-Chlor-thien-2-yl, 2-Methyl-thien-4-yl oder 1-Methyl-benzimidazol-2-yl und

$R^3$ und $R^4$ unabhängig voneinander Wasserstoff, Halogen, Nitro, Alkyl oder Halogenalkyl mit 1 bis 4 Kohlenstoffatomen, Cycloalkyl mit 3 bis 6 Kohlenstoffatomen, Alkoxy oder Alkylthio mit 1 bis 9 Kohlenstoffatomen, Thiophenyl, Amino, Alkylamino oder Dialkylamino mit 1 bis 4 Kohlenstoffatomen in einer Alkylgruppe, Aminoalkyl mit 1 bis 4 Kohlenstoffatomen, Alkylaminoalkyl oder Dialkylaminoalkyl mit 1 bis 4 Kohlenstoffatomen in einer Alkylgruppe, Alkanoylamino oder Halogenalkanoylamino mit 2 bis 5 Kohlenstoffatomen, Dialkylcarbamido mit 1 bis 4 Kohlenstoffatomen in einer Alkylgruppe, Alkoxycarbonylalkyl mit 3 bis 6 Kohlenstoffatomen, gegebenenfalls durch Alkoxy mit 1 bis 4 Kohlenstoffatomen substituiertes Alkoxycarbonyl mit 2 bis 6 Kohlenstoffatomen, Sulfo, Sulfino, Alkylsulfonyl oder Alkylsulfinyl mit 1 bis 4 Kohlenstoffatomen, Chlorsulfonyl, Phenylsulfonyl, Sulfamoyl, Alkylaminosulfinyl oder Alkylaminosulfonyl mit 1 bis 4 Kohlenstoffatomen, Hydroxyalkylamino- oder Di-hydroxyalkylaminosulfonyl mit 1 bis 4 Kohlenstoffatomen in einer Alkylgruppe, Morpholinyl-sulfonyl, Alkylsulfonylamino mit 1 bis 4 Kohlenstoffatomen, Alkoxycarbonylalkylamino mit 3 bis 6 Kohlenstoffatomen, N-(ω-Dialkylamino-alkyl)-amino-sulfonyl mit 1 bis 4 Kohlenstoffatomen in einer Alkylgruppe oder N-(2-Sulfoethyl)-amino-sulfonyl bedeuten,

mit der Maßgabe, daß $R^3$ Fluor, Nitro, Alkyl mit 2 bis 9 Kohlenstoffatomen, Halogenalkyl, Cycloalkyl, Alkoxy mit 4 bis 9 Kohlenstoffatomen, Alkylthio, Thiophenyl, Amino, Alkylamino, Dialkylamino, Alkanoylamino, Halogenalkanoylamino, Sulfo, Sulfino, Alkylsulfonyl, Alkylsulfinyl, Chlorsulfonyl, Phenylsulfonyl, Sulfamoyl, Alkylaminosulfinyl, Alkylaminosulfonyl, Hydroxyalkylaminosulfonyl, Di-hydroxyalkylaminosulfonyl, Morpholinylsulfonyl, Alkylsulfonylamino oder Alkoxycarbonylalkylamino bedeutet und

$R^4$ die obengenannten Bedeutungen hat, wenn $R^1$ Wasserstoff, Methyl, Ethyl, Halogenmethyl, Aminomethyl, Alkylaminomethyl oder Dialkylaminomethyl und $R^2$ Wasserstoff, Methyl oder Ethyl bedeuten, dadurch gekennzeichnet, daß man ein Anthranilsäurederivat der Formel

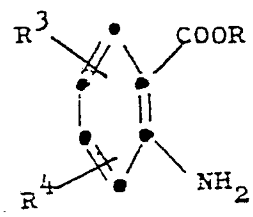

(II)

in der

R Wasserstoff oder Alkyl bedeutet und

$R^3$ und $R^4$ die oben genannten Bedeutungen haben, mit einem Thiocyanatderivat der Formel

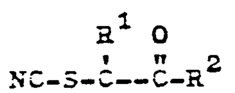

(III)

in der $R^1$ und $R^2$ die oben genannten Bedeutungen haben, oder mit einem Thiazolderivat der Formel

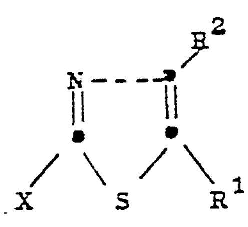

(IV)

in der

X Fluor, Chlor, Brom, Alkylsulfonyl oder Arylsulfonyl bedeutet und

$R^1$ und $R^2$ die oben genannten Bedeutungen haben,

umsetzt.

**Claims** (for the Contracting States : BE, CH, DE, FR, GB, IT, LI, NL)

1. A thiazolo [2,3-b]-quinazolone of the formula

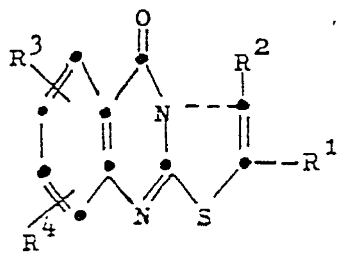

(I)

where

$R^1$ and $R^2$ independently of one another are hydrogen, halogen, nitro, alkyl of 1 to 12 carbon atoms, haloalkyl of 1 to 4 carbon atoms, cycloalkyl of 3 to 6 carbon atoms, alkoxy or alkylthio of 1 to 9 carbon atoms, thiophenyl, amino, alkylamino or dialkylamino, where alkyl is of 1 to 4 carbon atoms, aminoalkyl of 1 to 4 carbon atoms, alkylaminoalkyl or dialkylaminoalkyl, where alkyl is of 1 to 4 carbon atoms, alkanoylamino or haloalkanoylamino of 2 to 5 carbon atoms, dialkylcarbamido, where alkyl is of 1 to 4 carbon atoms, alkoxycarbonylalkyl of 3 to 6 carbon atoms, unsubstituted or $C_1$-$C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or di-(hydroxyalkyl)-aminosulfonyl, where hydroxyalkyl is of 1 to 4 carbon atoms, morpholinylsulfonyl, alkylsulfonylamino of 1 to 4 carbon atoms, alkoxycarbonylalkylamino of 3 to 6 carbon atoms, or are unsubstituted or halogen- or $C_1$-$C_4$-alkyl-monosubstituted thien-2-yl, thiazol-2-yl, fur-2-yl, benzimidazol-2-yl,2-chlorothien-2-yl, 2-methylthien-4-yl or 1-methylbenzimidazol-2-yl, and

$R^3$ and $R^4$ independently of one another are hydrogen, halogen, nitro, alkyl or haloalkyl of 1 to 4 carbon atoms, cycloalkyl of 3 to 6 carbon atoms, alkoxy or alkylthio of 1 to 9 carbon atoms, thiophenyl, amino, alkylamino or dialkylamino, where alkyl is of 1 to 4 carbon atoms, aminoalkyl of 1 to 4 carbon atoms, alkylaminoalkyl or dialkylaminoalkyl, where alkyl is of 1 to 4 carbon atoms, alkanoylamino or haloalkanoylamino of 2 to 5 carbon atoms, dialkylcarbamido, where alkyl is of 1 to 4 carbon atoms, alkoxycarbonylalkyl of 3 to 6 carbon atoms, unsubstituted or $C_1$-$C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or di(hydroxyalkyl)-aminosulfonyl, where alkyl is of 1 to 4 carbon atoms, morpholinylsulfonyl, alkylsulfonylamino of 1 to 4 carbon atoms, alkoxycarbonylalkylamino of 3 to 6 carbon atoms, N-(ω-dialkylaminoalkyl)-aminosulfonyl, where alkyl is of 1 to 4 carbon atoms, or N-(2-sulfoethyl)-aminosulfonyl, with the proviso that $R^3$ is fluorine, nitro, alkyl of 2 to 9 carbon atoms, haloalkyl, cycloalkyl, alkoxy of 4 to 9 carbon atoms, alkylthio, thiophenyl, amino, alkylamino, dialkylamino, alkanoylamino, haloalkanoylamino, sulfo, sulfino, alkylsulfonyl, alkylsulfinyl, chlorosulfonyl, phenylsulfonyl, sulfamyl, alkylaminosulfinyl, alkylaminosulfonyl, hydroxyalkylaminosulfonyl, di-(hydroxyalkyl)-aminosulfonyl, morpholinylsulfonyl, alkylsulfonylamino or alkoxycarbonylalkylamino, and

$R^4$ has the above meanings, when $R^1$ is hydrogen, methyl, ethyl, halomethyl, aminomethyl, alkylaminomethyl or dialkylaminomethyl, and $R^2$ is hydrogen, methyl or ethyl, or an acid addition salt of this compound.

2. A thiazolo [2,3-b]-quinazolone of the formula

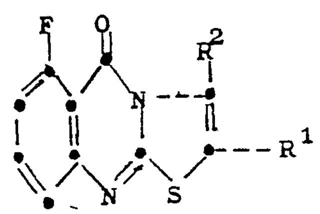

where $R^1$ is hydrogen, halogen, alkyl of 1 to 4 carbon atoms or alkoxycarbonyl of 2 to 5 carbon atoms, and $R^2$ is hydrogen, chloro, alkyl of 1 to 4 carbon atoms or alkoxycarbonyl of 2 to 5 carbon atoms, or an acid addition salt of this thiazolo [2,3-b]-quinazolone.

3. A thiazolo [2,3-b]-quinazolone of the formula

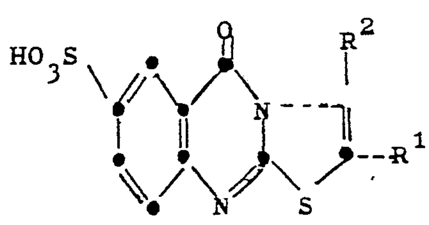

where $R^1$ is hydrogen, halogen, alkyl of 1 to 4 carbon atoms or alkoxycarbonyl of 2 to 5 carbon atoms, and $R^2$ is hydrogen, chloro, alkyl of 1 to 4 carbon atoms or alkoxycarbonyl of 2 to 5 carbon atoms, or an acid addition salt of this thiazolo [2,3-b]-quinazolone.

4. A process for the preparation of a thiazolo [2,3-b]-quinazolone of the formula I as claimed in claim 1, wherein an anthranilic acid derivative of the formula

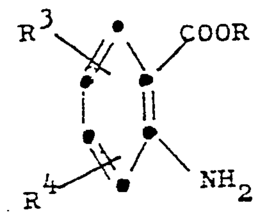

(II)

where

R is hydrogen or alkyl, and

$R^3$ and $R^4$ have the meanings given in claim 1, is reacted with a thiocyanate derivative of the formula

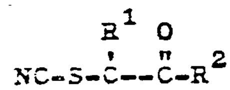

(III)

where $R^1$ and $R^2$ have the meanings given in claim 1, or with a thiazole derivative of the formula

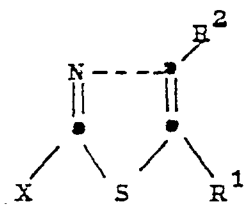

(IV)

where

X is fluorine, chlorine, bromine, alkylsulfonyl or arylsulfonyl, and

$R^1$ and $R^2$ have the meanings given in claim 1.

**Claims** (for the Contracting State AT)

A process for the preparation of a thiazolo [2,3-b]-quinazolone of the formula

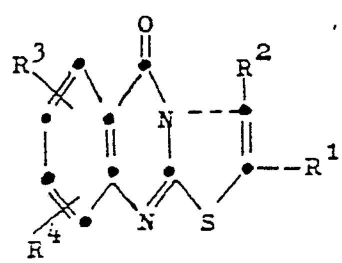

(I)

where

$R^1$ and $R^2$ independently of one another are hydrogen, halogen, nitro, alkyl of 1 to 12 carbon atoms, haloalkyl of 1 to 4 carbon atoms, cycloalkyl of 3 to 6 carbon atoms, alkoxy or alkylthio of 1 to 9 carbon atoms, thiophenyl, amino, alkylamino or dialkylamino, where alkyl is of 1 to 4 carbon atoms, aminoalkyl of 1 to 4 carbon atoms, alkylaminoalkyl or dialkylaminoalkyl, where alkyl is of 1 to 4 carbon atoms, alkanoylamino or haloalkanoylamino of 2 to 5 carbon atoms, dialkylcarbamido, where alkyl is of 1 to 4 carbon atoms, alkoxycarbonylalkyl of 3 to 6 carbon atoms, unsubstituted or $C_1$-$C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or di-(hydroxyalkyl)-aminosulfonyl, where hydroxyalkyl is of 1 to 4 carbon atoms, morpholinylsulfonyl, alkylsulfonylamino of 1 to 4 carbon atoms, alkoxycarbonylalkylamino of 3 to 6 carbon atoms, or are unsubstituted or halogen- or $C_1$-$C_4$-alkyl-monosubstituted thien-2-yl, thiazol-2-yl, fur-2-yl, benzimidazol-2-yl, 2-chlorothien-2-yl, 2-methylthien-4-yl or 1-methylbenzimidazol-2-yl, and

$R^3$ and $R^4$ independently of one another are hydrogen, halogen, nitro, alkyl or haloalkyl of 1 to 4 carbon atoms, cycloalkyl of 3 to 6 carbon atoms, alkoxy or alkylthio of 1 to 9 carbon atoms, thiophenyl, amino, alkylamino or dialkylamino, where alkyl is of 1 to 4 carbon atoms, aminoalkyl of 1 to 4 carbon atoms, alkylaminoalkyl or dialkylaminoalkyl, where alkyl is of 1 to 4 carbon atoms, alkanoylamino or haloalkanoylamino of 2 to 5 carbon atoms, dialkylcarbamido, where alkyl is of 1 to 4 carbon atoms, alkoxycarbonylalkyl of 3 to 6 carbon atoms, unsubstituted or $C_1$-$C_4$-alkoxy-substituted alkoxycarbonyl of 2 to 6 carbon atoms, sulfo, sulfino, alkylsulfonyl or alkylsulfinyl of 1 to 4 carbon atoms, chlorosulfonyl, phenylsulfonyl, sulfamyl, alkylaminosulfinyl or alkylaminosulfonyl of 1 to 4 carbon atoms, hydroxyalkylaminosulfonyl or di-(hydroxyalkyl)-aminosulfonyl, where alkyl is of 1 to 4 carbon atoms, morpholinylsulfonyl, alkylsulfonylamino of 1 to 4 carbon atoms, alkoxycarbonylalkylamino of 3 to 6 carbon atoms, N-(ω-dialkylaminoalkyl)-aminosulfonyl, where alkyl is of 1 to 4 carbon atoms, or N-(2-sulfoethyl)-aminosulfonyl,

with the proviso that $R^3$ is fluorine, nitro, alkyl of 2 to 9 carbon atoms, haloalkyl, cycloalkyl, alkoxy of 4 to 9 carbon atoms, alkylthio, thiophenyl, amino, alkylamino, dialkylamino, alkanoylamino, haloalkanoylamino, sulfo, sulfino, alkylsulfonyl, alkylsulfinyl, chlorosulfonyl, phenylsulfonyl, sulfamyl, alkylaminosulfinyl, alkylaminosulfonyl, hydroxyalkylaminosulfonyl, di-(hydroxyalkyl)-aminosulfonyl, morpholinylsulfonyl, alkylsulfonylamino or alkoxycarbonylalkylamino and

$R^4$ has the above meanings, when $R^1$ is hydrogen, methyl, ethyl, halomethyl, aminomethyl, alkylaminomethyl or dialkylaminomethyl, and $R^2$ is hydrogen, methyl or ethyl, wherein an anthranilic acid derivative of the formula

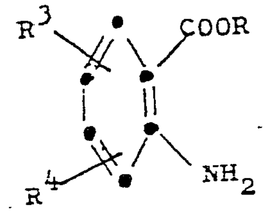

(II)

where

R is hydrogen or alkyl, and

$R^3$ and $R^4$ have the above meanings, is reacted with a thiocyanate derivative of the formula

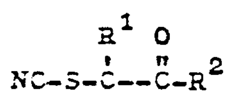

(III)

where $R^1$ and $R^2$ have the above meanings, or with a thiazole derivative of the formula

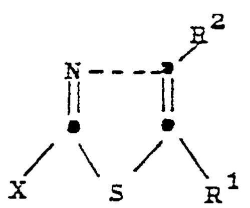

(IV)

where

X is fluorine, chlorine, bromine, alkylsulfonyl or arylsulfonyl, and

$R^1$ and $R^2$ have the above meanings.

**Revendications** (pour les Etats contractants : BE, CH, DE, FR, GB, IT, LI, NL)

1. Thiazolo [2,3-b] quinazolones de formule

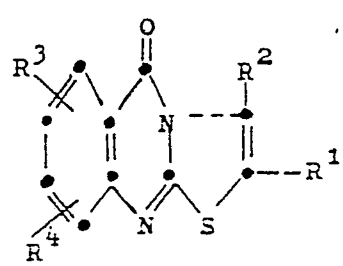

(I)

dans laquelle

$R^1$ et $R^2$ représentent, indépendamment l'un de l'autre, hydrogène, halogène, nitro, alkyle de 1 à 12 atomes de carbone, halogénalkyle de 1 à 4 atomes de carbone, cycloalkyle de 3 à 6 atomes de carbone, alcoxy ou alkylthio de 1 à 9 atomes de carbone, thiophényle, amino, alkylamino ou dialkylamino de 1 à 4 atomes de carbone dans un groupe alkyle, aminoalkyle de 1 à 4 atomes de carbone, alkylaminoalkyle ou dialkylaminoalkyle de 1 à 4 atomes de carbone dans un groupe alkyle, alcanoylamino ou halogénalcanoylamino de 2 à 5 atomes de carbone, dialkylcarbamido de 1 à 4 atomes de carbone dans un groupe alkyle, alcoxycarbonylalkyle de 3 à 6 atomes de carbone, alcoxycarbonyle de 2 à 6 atomes de carbone, éventuellement substitué par alcoxy de 1 à 4 atomes de carbone, sulfo, sulfino, alkylsulfonyle ou alkylsulfinyle de 1 à 4 atomes de carbone, chlorosulfonyle, phénylsulfonyle, sulfamoyle, alkylaminosulfinyle ou alkylaminosulfonyle de 1 à 4 atomes de carbone, hydroxyalkylaminosulfonyle ou dihydroxyalkylaminosulfonyle de 1 à 4 atomes de carbone dans un groupe hydroxyalkyle, morpholinyl-sulfonyle, alkylsulfonylamino de 1 à 4 atomes de carbone, alcoxycarbonylalkylamino de 1 à 6 atomes de carbone, ou thién-2-yle éventuellement substitué une fois par halogène ou alkyle de 1 à 4 atomes de carbone, thiazol-2-yle, fur-2-yle, benzimidazol-2-yle, 2-chloro-thién-2-yle, 2-méthyl-thién-4-yle ou 1-méthylbenzimidazol-2-yl,

et $R^3$ et $R^4$ représentent, indépendamment l'un de l'autre, hydrogène, halogène, nitro, alkyle ou halogénalkyle de 1 à 4 atomes de carbone, cycloalkyle de 3 à 6 atomes de carbone, alcoxy ou alkylthio de 1 à 9 atomes de carbone, thiophényle, amino, alkylamino ou dialkylamino de 1 à 4 atomes de carbone dans un groupe alkyle, aminoalkyle de 1 à 4 atomes de carbone, alkylaminoalkyle ou dialkylaminoalkyle de 1 à 4 atomes de carbone dans un groupe alkyle, alcanoylamino ou halogénalcanoylamino de 2 à 5 atomes de carbone, dialkylcarbamido de 1 à 4 atomes de carbone dans un groupe alkyle, alcoxycarbonylalkyle de 3 à 6 atomes de carbone, alcoxycarbonyle de 2 à 6 atomes de carbone, éventuellement substitué par alcoxy de 1 à 4 atomes de carbone, sulfo, sulfino, alkylsulfonyle ou alkylsulfinyle de 1 à 4 atomes de carbone, chlorosulfonyle, phénylsulfonyle, sulfamoyle, alkylaminosulfinyle ou alkylaminosulfonyle de 1 à 4 atomes de carbone, hydroxyalkylamino- ou di-hydroxyalkylaminosulfonyle de 1 à 4 atomes de carbone dans un groupe alkyle, morpholinyl-sulfonyle, alkylsulfonylamino de 1 à 4 atomes de carbone, alcoxycarbonylalkylamino de 3 à 6 atomes de carbone, N-(ω-dialkylamino-alkyl)-aminosulfonyle de 1 à 4 atomes de carbone dans un groupe alkyle ou N-(2-sulfo-éthyl)-amino-sulfonyle,

sous réserve que $R^3$ représente fluor, nitro, alkyle de 2 à 9 atomes de carbone, halogénalkyle, cycloalkyle, alcoxy de 4 à 9 atomes de carbone, alkylthio, thiophényle, amino, alkylamino, dialkylamino, alcanoylamino, halogénalcanoylamino, sulfo, sulfino, alkylsulfonyle, alkylsulfinyle, chlorosulfonyle, phénylsulfonyle, sulfamoyle, alkylaminosulfinyle, alkylaminosulfonyle, hydroxyalkylaminosulfonyle, di-hydroxyalkylaminosulfonyle, morpholinylsulfonyle, alkylsulfonyleamino ou alcoxycarbonylalkylamino, et

$R^4$ a les significations données plus haut lorsque $R^1$ est hydrogène, méthyle, éthyle, halogénométhyle, aminométhyle, alkylaminométhyle ou dialkylaminométhyle et $R^2$ représente hydrogène, méthyle ou éthyle, ou des sels d'addition d'acide de ces composés.

2. Thiazolo [2,3-b]-quinazolones de formule

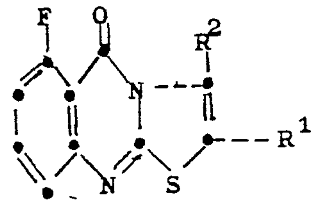

dans laquelle $R^1$ représente hydrogène, halogène, alkyle de 1 à 4 atomes de carbone ou alcoxycarbonyl de 2 à 5 atomes de carbone et $R^2$ est hydrogène, chlore, alkyle de 1 à 4 atomes de carbone ou alcoxycarbonyle de 2 à 5 atomes de carbone, ainsi que des sels d'addition d'acide desdites thiazole [2,3-b]-quinazolone.

3. Thiazolo [2,3-b] quinazolone de formule

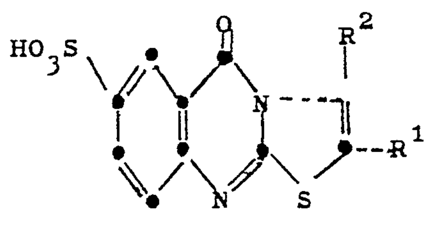

dans laquelle $R^1$ représente hydrogène, halogène, alkyle de 1 à 4 atomes de carbone ou alcoxycarbonyle de 2 à 5 atomes de carbone, $R^2$ étant hydrogène, chlore, alkyle de 1 à 4 atomes de carbone ou alcoxycarbonyle de 2 à 5 atomes de carbone, ainsi que des sels d'addition d'acide desdites thiazolo-[2,3-b]-quinazolones.

4. Procédé de préparation de thiazolo [2,3-b]-quinazolone de formule I selon la revendication 1, caractérisé en ce qu'on fait réagir un dérivé d'acide anthranilique de formule

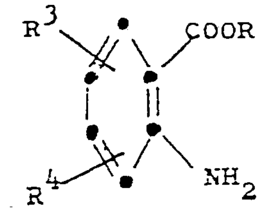

(II)

dans laquelle R représente hydrogène ou alkyle,

$R^3$ et $R^4$ ayant les significations données dans la revendication 1, avec un dérivé de thiocyanate de formule

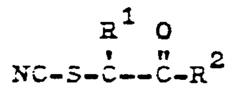

(III)

dans laquelle $R^1$ et $R^2$ ont les significations données dans la revendication 1, ou avec un dérivé de thiazole de formule

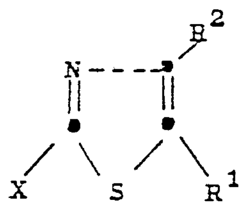

(IV)

dans laquelle

X représente fluor, chlore, brome, alkylsulfonyle ou arylsulfonyle,

$R^1$ et $R^2$ ayant les significations données dans la revendication 1.

**Revendication** (pour l'Etat contractant AT)

Procédé de préparation de thiazolo [2,3-b] quinazolones de formule

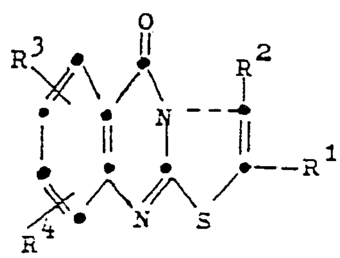

(I)

dans laquelle

$R^1$ et $R^2$ représentent, indépendamment l'un de l'autre, hydrogène, halogène, nitro, alkyle de 1 à 12 atomes de carbone, halogénalkyle de 1 à 4 atomes de carbone, cycloalkyle de 3 à 6 atomes de carbone, alcoxy ou alkylthio de 1 à 9 atomes de carbone, thiophényle, amino, alkylamino ou dialkylamino de 1 à 4 atomes de carbone dans un groupe alkyle, aminoalkyle de 1 à 4 atomes de carbone, alkylaminoalkyle ou dialkylaminoalkyle de 1 à 4 atomes de carbone dans un groupe alkyle, alcanoylamino ou halogénalcanoylamino de 2 à 5 atomes de carbone, dialkylcarbamido de 1 à 4 atomes de carbone dans un groupe alkyle, alcoxycarbonylalkyle de 3 à 6 atomes de carbone, alcoxycarbonyle de 2 à 6 atomes de carbone, éventuellement substitué par alcoxy de 1 à 4 atomes de carbone, sulfo, sulfino, alkylsulfonyle ou alkylsulfinyle de 1 à 4 atomes de carbone, chlorosulfonyle, phénylsulfonyle, sulfamoyle, alkylaminosulfinyle ou alkylaminosulfonyle de 1 à 4 atomes de carbone, hydroxyalkylaminosulfonyle ou dihydroxyalkylaminosulfonyle de 1 à 4 atomes de carbone dans un groupe hydroxyalkyle, morpholinyl-sulfonyle, alkylsulfonylamino de 1 à 4 atomes de carbone, alcoxycarbonylalkylamino de 1 à 6 atomes de carbone, ou thién-2-yle éventuellement substitué une fois par halogène ou alkyle de 1 à 4 atomes de carbone, thiazol-2-yle, fur-2-yle, benzimidazol-2-yle, 2-chloro-thién-2-yle, 2-méthyl-thién-4-yle ou 1-méthylbenzimidazol-2-yl,

et $R^3$ et $R^4$ représentent, indépendamment l'un de l'autre, hydrogène, halogène, nitro, alkyle ou halogénalkyle de 1 à 4 atomes de carbone, cycloalkyle de 3 à 6 atomes de carbone, alcoxy ou alkylthio de 1 à 9 atomes de carbone, thiophényle, amino, alkylamino ou dialkylamino de 1 à 4 atomes de carbone dans un groupe alkyle, aminoalkyle de 1 à 4 atomes de carbone, alkylaminoalkyle ou dialkylaminoalkyle de 1 à 4 atomes de carbone dans un groupe alkyle, alcanoylamino ou halogénalcanoylamino de 2 à 5 atomes de carbone, dialkylcarbamido de 1 à 4 atomes de carbone dans un groupe alkyle, alcoxycarbonylalkyle de 3 à 6 atomes de carbone, alcoxycarbonyle de 2 à 6 atomes de carbone, éventuellement substitué par alcoxy de 1 à 4 atomes de carbone, sulfo, sulfino, alkylsulfonyle ou alkylsulfinyle de 1 à 4 atomes de carbone, chlorosulfonyle, phénylsulfonyle, sulfamoyle, alkylaminosulfinyle ou alkylaminosulfonyle de 1 à 4 atomes de carbone, hydroxyalkylamino- ou di-hydroxyalkylamino-sulfonyle de 1 à 4 atomes de carbone dans un groupe alkyle, morpholinyl-sulfonyle, alkylsulfonylamino de 1 à 4 atomes de carbone, alcoxycarbonylalkylamino de 3 à 6 atomes de carbone, N-(ω-dialkylamino-alkyl)-aminosulfonyle de 1 à 4 atomes de carbone dans un groupe alkyle ou N-(2-sulfoéthyl)-amino-sulfonyle,
sous réserve que $R^3$ représente fluor, nitro, alkyle de 2 à 9 atomes de carbone, halogénalkyle, cycloalkyle, alcoxy de 4 à 9 atomes de carbone, alkylthio, thiophényle, amino, alkylamino, dialkylamino, alcanoylamino, halogénalcanoylamino, sulfo, sulfino, alkylsulfonyle, alkylsulfinyle, chlorosulfonyle, phénylsulfonyle, sulfamoyle, alkylaminosulfinyle, alkylaminosulfonyle, hydroxyalkylaminosulfonyle, di-hydroxyalkylaminosulfonyle, morpholinylsulfonyle, alkylsulfonyleamino ou alcoxycarbonylalkylamino, et

$R^4$ a les significations données plus haut lorsque $R^1$ est hydrogène, méthyle, éthyle, halogénométhyle, aminométhyle, alkylaminométhyle ou dialkylaminométhyle et $R^2$ représente hydrogène, méthyle ou éthyle, caractérisé par le fait qu'on fait réagir un dérivé anthranilique de formule

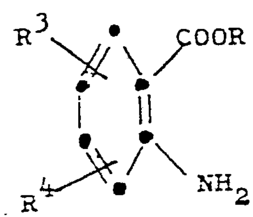

(II)

dans laquelle

R représente hydrogène ou alkyle,

$R^3$ et $R^4$ ayant les significations indiquées ci-dessus, avec un dérivé de thiocyanate de formule

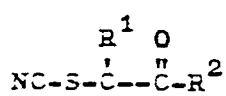

(III)

dans laquelle $R^1$ et $R^2$ ont les significations indiquées ci-dessus, ou avec un dérivé de thiazole de formule

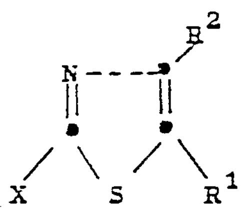

(IV)

dans laquelle

X représente fluor, chlore, brome, alkylsulfonyle ou arylsulfonyle,

et $R^1$ et $R^2$ ayant les significations indiquées ci-dessus.